# EUROPEAN PATENT SPECIFICATION

(11) **EP 1 734 933 B1**
(45) Date of publication and mention of the grant of the patent: **26.05.2010**
(21) Application number: 05733297.5
(22) Date of filing: 24.03.2005
(51) Int. Cl.: A61K 9/26, A61K 9/58, A61K 31/403

(54) **GASTRORESISTANT PHARMACEUTICAL DOSAGE FORM COMPRISING N-(2-(2-PHTHALIMIDOETHOXY)-ACETYL)-L-ALANYL-D-GLUTAMIC ACID (LK-423)**
MAGENRESISTENTE PHARMAZEUTISCHE DOSIERFORM MIT N-(2-(2-PHTHALIMIDOETHOXY)-ACETYL)-L-ALANYL-D-GLUTAMINSÄURE (LK-423)
FORME GALENIQUE PHARMACEUTIQUE GASTRORESISTANTE COMPRENANT L'ACIDE N-(2-(2-PHTALIMIDOETHOXY)-ACETYL)-L-ALANYL-D-GLUTAMIQUE (LK-423)

(30) Priority: 26.03.2004 SI 200400094
(43) Date of publication of application: 27.12.2006
(73) Proprietor: LEK Pharmaceuticals d.d., 1526 Ljubljana (SI)
(72) Inventor: BOGATAJ, Marija, 1370 Logatec (SI); MRHAR, Ales, 1000 Ljubljana (SI); LAVRIC, Anton, 1291 Skofljica (SI); CERNE, Manica, 1292 Ig (SI); TIBAUT, Doris, 1000 Ljubljana (SI); STALC, Anton, 1000 Ljubljana (SI); URLEB, Uros, 1000 Ljubljana (SI); MATEOVIC, Tatjana, 1000 Ljublijana (SI); COF, Greta, 1215 Medvode (SI); KERK, Janez, 1000 Ljubljana (SI); DREU, Rok, 2380 Slovenj Gradec (SI); YONEDA, Fumio, Takatsuki City, Osaka 569-1020 (JP); MURAOKA, Shizuko, Matsubara City, Osaka 580-0016 (JP)
(86) International application number: PCT/EP2005/003175
(87) International publication number: WO 2005/092295

(56) References cited:
- EP-A- 0 477 912
- MORIGUCHI M ET AL: "THERAPEUTIC EFFECTS OF LK 423, A PHTHALIMIDO-DESMURAMYL-DIPEPTIDE COMPOUND, ON DEXTRAN SULFATE SODIUM INDUCED COLITIS IN RODENTS THROUGH RESTORING THEIR INTERLEUKIN-10 PRODUCING CAPACITY" ARZNEIMITTEL FORSCHUNG. DRUG RESEARCH, EDITIO CANTOR VERLAG, AULENDORF, DE, vol. 49, no. 1, 1999, pages 184-192, XP002924596 ISSN: 0004-4172 cited in the application
- OCHI C ET AL: "INTERLEUKIN-10 INDUCING ACTIVITY OF LK423, A PHTHALIMIDO-DESMURAMYLDIPEPTIDE COMPOUND" ARZNEIMITTEL FORSCHUNG. DRUG RESEARCH, EDITIO CANTOR VERLAG, AULENDORF, DE, vol. 49, no. 1, 1999, pages 72-79, XP002924595 ISSN: 0004-4172 cited in the application

## Description

### Field of the invention

The present invention relates to pharmaceutical dosage forms which can be used for controlled and/or targeted delivery of the active substance to the selected regions of gastrointestinal tract of humans or animals. The pharmaceutical dosage forms of the present invention will be mainly described by referring to the active substance N-(2-(2-phthalimidoethoxy)-acetyl)-L-alanyl-D-glutamic acid (designated as LK 423) although the pharmaceutical dosage forms of the present invention can be applied for other active substances.
A further aspect of the present invention relates to methods and processes for obtaining said pharmaceutical dosage forms and the methods of treatment of chronic inflammatory diseases of humans and/or animals by using the pharmaceutical dosage forms of the present invention.

### Background of the invention

N-(2-(2-phthalimidoethoxy)-acetyl)-L-alanyl-D-glutamic acid (designated as LK 423) was described in EP 477 912 (US 5,514,654). Its effects on the regulation of cytokine production, inhibition of anti-inflammatory cytokine production, and stimulation of cytokine production, in particular of IL-10 are described in WO 0135982 and in scientific literature (Ochi C. et al. Arzneimittel-Forschung, (1999) 49(1): 72-79; Moriguchi M. et al, Arzneimittel-Forschung (1999) 49(2): 184-192). The effects of LK 423 in the treatment of a variety of inflammatory diseases in particular for the treatment of ulcerative colitis and/or Chron's disease were disclosed in WO 0135982. The possibility to prepare pharmaceutical compositions for oral and non-oral administration of the active substance LK 423 was mentioned in WO 0135982. However, the detailed description of pharmaceutical dosage forms comprising LK 423 has not been provided either in the patent or in the scientific literature.

The use of pharmaceutical dosage forms which effectively deliver the active substance to the selected regions of the gastrointestinal tract (*e.g*. colon) where the active substance is released is the most reasonable for the treatment of local diseases (*i.e.* ulcerative colitis and Chron's disease) and for the systemic treatment with the active substances which easily degrade in the upper segments of the gastrointestinal tract (protein and peptide active substances). Since the access to the distal segments of the gastrointestinal tract is rather complicated and difficult the dosage forms for delivery of the active substances to these segments are complex. Despite a difficult access to the colon, several possible approaches in colon-specific drug delivery have been developed (Rubinstein A., Crit Rev Ther Drug Carrier Syst, 12 (2&3), (1995) 101-149). In Friend D.R., Adv Drug Deliv Rev, 7, (1991) 149-199, two goals in designing pharmaceutical dosage forms for colonic targeting are:
- delivery of the unchanged active substance through the stomach and the small intestine to the colon;
- specific release of the active substance in the colon with satisfactory predictability and reproducibility.

These goals can be applied for delivery of the drug to other distal portions of the gastrointestinal tract of humans and animals.

Different approaches for controlled and / or selective and / or targeted delivery of drugs into gastrointestinal tract are described in the patent literature: EP 527942 (WO 9116881, US 5525634 and US 586661); WO 9200732; EP 366621 (US 5171580 and WO 9004386); EP 553392; EP 636366 (US 5286493 and US 5580578); EP 463877; EP 453001; WO 9116042; US 5840332; GB 1219026; WO 8300435; EP 40590; EP 225189; US 2002/0051818 (US 2001/0005716, US 2001/0024660); EP 810857 (US 6228396; WO 9535100) and US 5350741 and in the scientific literature: Gruber P et al, Ad Drug Del Rev (1987) 1: 1-18; Rao S and Ritschel WA, S.T.P. Pharma Sci 1995; 5: 19-29; Evans DF et al Gut (1988) 29: 1035-1041; Lee VHL et al Peptide and protein drug delivery, Lee VHL Ed., Marcel Dekker New York, (1990) 712-720; Rubinstein A. Crit Rev Ther Drug Carrier Syst, 12 (2&3), (1995) 101-149; Wilding IR et al Pharmac Ther (1994) 62: 97-124; Peeters R and Kinget R, Int J Pharm (1993) 94: 125-134; Evans DF et al Gut (1988) 29: 1035-1041; Ashford M et al, Int J Pharm (1993) 91: 241-245; Ashford M et al Int J Pharm (1993) 95: 193-199; Follonier N and Doelker E, S.T.P. Pharma Sci (1992) 2: 141-158; Hwang SJ et al Crit Rev Ther Drug Carrier Syst (1998) 15: 243-284; Coupe AJ et al Pharm Res (1991) 8: 360-364; Mooter van den G and Kinget R, Drug Del (1995) 2: 81-93; Wilding IR et al Pharmac Ther (1994) 62: 97-124; Wilding IR et al Int J Pharm (1994) 111: 99-102; Fukui E et al Int J Pharm (2000) 204: 7-15; Gazzaniga A. et al Pharma Sci, 5(1), (1995) 83-88; Ishibashi T. et al Int J Pharm, 168, (1998) 31-40; Ishibashi T. et al J Control Release, 57, (1999) 45-53; Kraeling M.E.K. and Ritschel W.A., Methods Find Exp Clin Pharmacol, 14(3), (1992) 199-209; Ashford M. et al J Controlled Release (1993) 26: 213-220; Ashford M. et al J Controlled Release (1994) 30: 225-232; Rubinstein A et al Pharm Res (1993) 10: 258-263; Sriamornsak, P. Int. J. Pharm. (1998) 169: 213-220; Sriamornsak, P. and Nunthanid, J. Int. J. Pharm. (1998) 160: 207-212; Sriamornsak, P., Puttipipatkhachorn, S., Prakongpan, S., Int. J. Pharm. (1997) 156: 189-194; Sriamornsak, P. et al J. Controlled Release (1997) 47: 221-232; Sriamornsak, P., Int. J. Pharm. (1998) 169: 213-220; Wakerly, Z. et al Pharm. Res. (1996) 13: 1210-1212; Macleod, G.S et al J. Controlled Release (1999) 58: 303-310; Macleod, G.S., et al Int. J. Pharm. (1999) 187: 251-257; Munjeri, O. et al J. Controlled Release (1997) 46: 273-278.

For the development of the drug delivery systems specific for delivery to distal portions of gastrointestinal tract (*e.g*. colon) parameters like the pH values (leading to the development of pH controlled systems), gastrointestinal transit time (leading to the time-controlled systems) and in the colon specifically degradable polymers e.g. degradable polysaccharides are important.

By specific delivery of the active substance to the selected segments of the gastrointestinal tract it is possible to achieve a high concentration of an active substance in the affected segment, and low systemic burden leading to few side effects. In comparison to a systemic delivery of an active substance a specific delivery enables smaller breakdown of the active substance, a greater extent of absorption, a better absorption profile and fewer local side effects.

In the field of the treatment of inflammatory bowel diseases, there is a constant need for pharmaceutical dosage forms which would enable controlled, targeted and effective administration and/or delivery of the active substance to the diseased region and which are simple and do not require assistance of the medicinal personnel for application, at the same time being economical, effective, safe with minimum side effects, and human or animal friendly.

### Summary of the invention

The object of the present invention are new pharmaceutical dosage forms which can be used for controlled and/or targeted delivery of the active substance to the gastrointestinal tract of humans and animals, preferably to the selected regions of the gastrointestinal tract. The pharmaceutical dosage forms of the present invention enable a controlled release of the active substance in the predetermined concentrations into the selected regions of the gastrointestinal tract, especially into the distal portions of the gastrointestinal tract of humans or animals. Although the pharmaceutical dosage forms of the present invention have been developed for the active substance N-(2-(2-phthalimidoethoxy)-acetyl)-L-alanyl-D-glutamic acid (designated as LK 423), the concept of the invention and the pharmaceutical dosage forms of the present invention can be applied for other active substances that need the controlled and/or targeted delivery to the selected regions of gastrointestinal tract.

The pharmaceutical dosage form of the present invention can be used for the treatment of chronic inflammatory diseases such as inflammatory bowel diseases in humans and animals.

The present invention provides a new pharmaceutical dosage form comprising the active substance in accordance with claim 1 and 18. Preferred embodiments are set forth in the sub-claims.

### Description of the figures

Figure 1 shows a schematic structure of some pharmaceutical dosage forms according to the invention.
Figure 2 shows the release profile of the active substance from the microcapsules of 1600-2000 µm in size, coated with 10% Eudragit® RS/Eudragit® RL mixture in the ratio 1:1 and 30% Eudragit® L-55 according to Example 1.
Figure 3 shows the release profile of the active substance from the microcapsules of 1250-2000 µm in size, coated with 3% Eudragit® RS/Eudragit® RL mixture in the ratio 1:1 and 30% Eudragit® L-55 according to Example 2.
Figure 4 shows the release profile of the active substance from the tablets consisting of single-coated microcapsules and compressed into tablets with hydroxypropylmethyl cellulose phthalate (HPMCP) / Kollidon^{®} SR mixture according to Example 3.
Figure 5 shows the intestinal mucosa with DSS-induced coagulation necrosis not treated with LK 423. Superficial and glandular epithelium missing in the area of necrosis. The propria of the mucosa is infiltrated with inflammatory cells. Large hemorrhage surrounded with inflammatory cells in the strongly edematous submucosa.
   Organ: colon. Staining - HE (hematoxylin - eosin). Magnification: 40x.
Figure 6 shows the necrotic propria of the mucosa with DSS (dextran sulphate sodium) -induced coagulation necrosis which was then treated directly with the pure compound LK 423. The propria of the mucosa is infiltrated with inflammatory cells (active inflammatory process), including many fibroblasts. A surface of the propria of the mucosa is covered with the epithelium which is of uneven thickness and detaches from the base. No renewal of the glandular epithelium in the propria is visible. Inflammatory cells and fibroblasts accumulate in the edematous submucosa under the lamina muscularis.
   Organ: colon. Staining - HE (hematoxylin - eosin). Magnification: 40x.
Figure 7 shows the necrotic propria of the mucosa with DSS-induced coagulation necrosis which was then treated with microcapsules comprising LK 423 that is in the pharmaceutical dosage form of the present invention. The granulation tissue is in the area of necrosis in the mucosa and submucoca. Covering of the granulation tissue in the mucosa by the superficial epithelium from the margins of the damage. *De-novo* formation of the correctly structured glands and lymph follicles. Acute hemorrhage and acute inflamed infiltration absent.
   Organ: colon. Staining - HE (hematoxylin - eosin). Magnification: 40x.

### Description of the invention

It was found that the pharmaceutical dosage forms of the present invention which comprise an active substance can be used for controlled and/or targeted delivery of the active substance to the selected regions of gastrointestinal tract, especially to the distal portions of gastrointestinal tract of humans and animals i.e. ileum, caecum and colon.
Preferably, the pharmaceutical dosage forms of the present invention comprise the active substance N-(2-(2-phthalimidoethoxy)-acetyl)-L-alanyl-D-glutamic acid (LK 423).

The pharmaceutical dosage forms of the present invention comprise a core, an inner coat and optionally an outer coat.

The term 'core' as used herein, refers to the central part of a pharmaceutical dosage form containing the active substance *per se* or containing the active substance incorporated into a polysaccharide matrix. Said core may be in the form of a spheroid particle which is selected from the group consisting of a microsphere, microparticle, pellet, granule or other analog and/or similar form.

The term 'coat' as used herein, refers to a layer surrounding the core.

The core is in the interior of the pharmaceutical dosage form of the present invention. The pharmaceutical dosage form may have more coats so that the core is in the interior, surrounded with one inner coat and optionally one outer coat.

The term 'inner coat' as used herein, refers to a coat which is applied directly to the core, and is interchangeable with the terms a retard coat, a retard type coat, a coat from retard polymers and a coat for sustained release.

The term 'subcoat' as used herein refers to a layer which is applied under inner or under outer coat to separate core from inner coat and / or inner coat from outer coat. It may be used when the incompatibilities could appear between the components of the core and components of inner coat and / or between the components of inner and outer coat.

The term 'outer coat' as used herein refers to a coat which is applied to the inner coat, and is interchangeable with the terms gastroresistant coat, enteric coat, acidoresistant coat, coat from gastroresistant and/or acidoresistant polymers.

In the pharmaceutical dosage form of the present invention the core comprises an active substance and a polysaccharide (forming a polysaccharide matrix) whereby the ratio of the active substance to a polysaccharide is from about 4:1 to about 1:4 (w/w). Preferably, the active substance is LK 423 and the ratio of LK 423 to a polysaccharide is about 1:1 (w/w). The polysaccharide is selected from the group consisting of pectin or alginate, either in the form of acid or in the form of metal salt, galactomannans, covalently crosslinked dextran, amylose, xanthans, carrageenan and starch or combinations of the said polysaccharides or their salts with the same specific degradability. Preferably, the selected polysaccharide is pectin, more preferably in the form of calcium salt (calcium pectinate).

Preferably, the core is a solid dispersion of the active substance LK 423 in the calcium pectinate, forming a calcium pectinate matrix. The calcium pectinate matrix is specifically degradable by bacterial flora in distal portions of the gastrointestinal tract. In the contact with an aqueous medium, it swells, forms hydrogel through which the active substance is released. The active substance in the matrix may be dissolved, suspended or partly dissolved and partly suspended. The solid dispersion is in the form of spherical beads.

Furthermore, the core preferably further comprises a glidant. The glidants may increase the homogeneity of suspensions in the process of core preparation. When the glidant is present, the proportion of the glidant is from about 15 to about 60% (w/w) to the weight of the cores. The glidant in the core is selected from the group consisting of magnesium stearate, calcium stearate or aerosil. Preferably, the glidants are magnesium stearate or aerosil.

The cores of the pharmaceutical dosage forms of the present invention have a diameter from about 100 µm to about 2800 µm.

The core of the pharmaceutical dosage form of the present invention is surrounded by inner coat which enables sustained release of the active substance from the core. The inner coat prevents release of the active substance in the proximal portions of the small intestine and enables its release in ileum, caecum and colon.

The inner coat comprises a polymer which is insoluble in water and is partly permeable for water and aqueous solutions. The polymer is selected from the group consisting of methacrylate ester copolymers, mixture of polyvinyl acetate (PVAc) and polyvinylpyrrolidone (PVP) and/or combinations thereof. The inner coat may comprise a combination of two or more polymers. When the inner coat comprises a combination of two polymers, both are pH-independent and insoluble in aqueous media. The combination of two polymers is selected from the group consisting of methacrylate ester copolymers (combination of copolymers of acrylic and methacrylic acid esters with a low content of quaternary ammonium groups) (e.g. Eudragit RS, Eudragit RL). Preferably, the inner coat comprises a combination of methacrylic and acrylic acid esters derivatives which are copolymers of acrylic and methacrylic acid esters with a low content of quaternary ammonium groups, the molar ratio of ammonium groups to remaining neutral esters being 1:20 in the first polymer, and 1:40 in the second polymer. More preferably, the first polymer is ammonio methacrylate copolymer type A according to USP/NF (*e.g*. Eudragit^{®} RL) and the second polymer is ammonio methacrylate copolymer type B according to USP/NF (*e.g.* Eudragit^{®} RS). Preferably, the copolymers are applied on the core in the form of dispersion, more preferably in the form of 30% aqueous dispersion.

The inner coat preferably further comprises other pharmaceutically acceptable excipients. Suitable pharmaceutically acceptable excipients are selected from the group consisting of a glidant, a plasticizer, an anti-foaming agent and a pigment.
A glidant of the inner coat is selected from the group consisting of hydrous magnesium silicate (talc), kaolin and glycerol monostearete. Preferably the selected glidant of the inner coat is talc.
A suitable plasticizer of the inner coat is selected from the group consisting of triethyl citrate, tributyl citrate, acetyltriethyl citrate, acetyltributyl citrate, diethyl phthalate, dibutyl phthalate, dibutyl sebacate, glyceryl triacetate, triacetin, polyethylene glycol 6000 and polyoxyethylene (20) sorbitan monooleate. Preferably the plasticizer of the inner coat is triethyl citrate.

In the pharmaceutical dosage form of the present invention having one core and one (inner) coat the proportion of the core is from about 50% to about 98% (w/w) and the proportion of the coat is from about 2% to about 50% (w/w) to the total weight of the pharmaceutical dosage form. Preferably the proportion of the core in said dosage form is from about 70 to about 97% by weight and the proportion of the coat from about 2% to about 30% by weight to the total weight of the pharmaceutical dosage form.

In the pharmaceutical dosage form of the present invention the outer coat may be applied to the inner coat. The outer coat is insoluble in an acidic environment at the pH below 5 and prevents release of the active substance in the acidic medium of the stomach. The outer coat preferably comprises a polymer soluble at pH above 5.5 *(i.e.* pH of small intestinal fluids).

The outer coat may comprise any acidoresistant polymer selected from the group consisting of derivatives of methacrylic acid copolymers (*e.g*. Eudragit^{®} L, Eudragit^{®} L-55, Eudragit^{®} S, Eudragit^{®} FS), HPMCP, hydroxyethylcellulose phthalate (HECP), cellulose acetate phthalate (CAP), polyvinyl acetyl phthalate (PAP), hydroxypropylmethylcellulose acetate succinate (HPMC AS) or combinations thereof. Preferably the outer coat comprises an anionic copolymer based on methacrylic acid and ethyl acrylate. According to USP/NF, a suitable polymer is methacrylic acid copolymer type C (*e.g*. Eudragit^{®} L-55) in the form of dispersion, more preferably in the form of the 30% aqueous dispersion.

The outer coat of the present invention may preferably further comprise other pharmaceutically acceptable excipients selected from the group consisting of a glidant, a plasticizer, an antifoaming agent and a pigment. Preferably, a glidant and a plasticizer are used.

A glidant of the outer coat is selected from the group consisting of talc, kaolin and glycerol monostearate. Preferably, the glidant of the outer coat is talc.

A plasticizer of the outer coat is selected from the group consisting of triethyl citrate, tributyl citrate, acetyltriethyl citrate, acetyltributyl citrate, diethyl phthalate, dibutyl phthalate, dibutyl sebacate, glyceryl triacetate, triacetin, polyethylene glycol 6000 and polyoxyethylene (20) sorbitan monooleate. Preferably, the plasticizer of the outer coat is triethyl citrate.

In the pharmaceutical dosage form of the present invention having a core, an inner and an outer coat, the proportion of the core is from about 30 to about 90% (w/w), the proportion of the inner coat is from about 2% to about 50% (w/w) and the proportion of the outer coat is from about 6 % to about 50% (w/w) to the total weight of the pharmaceutical dosage form. Preferably the proportion of the core in said dosage form is from about 55% to about 90% (w/w), the proportion of the inner coat from about 2 % to about 30% (w/w) and the proportion of the outer coat from about 6% to about 32% (w/w) to the total weight of the pharmaceutical dosage form.

The site and kinetic of release of the active substance from the pharmaceutical dosage form of the present invention can be adjusted by the core size, the content of the active substance and the thickness and composition of the inner coat and thickness and composition of the outer coats. In this respect the combinations of cores of different diameter and inner and outer coats of different thickness and compositions are possible. If the thickness of the inner coat and/or outer coat is greater, release of the active substance is retarded.

In the most preferred aspect of the invention the pharmaceutical dosage form suitable for controlled and/or targeted delivery of the active substance to the distal portions of the gastrointestinal tract comprises
- a core - a calcium pectinate matrix in which LK 423 is dispersed, comprising magnesium stearate as a glidant, and
- inner coat - comprising polymers Eudragit RS and Eudragit RL, talc as a glidant and triethyl citrate as a plasticizer, and
- outer coat - comprising polymer Eudragit L-55, talc as a glidant and triethyl citrate as a plasticizer.

The pharmaceutical dosage form of the present invention may be in a form of a microcapsule, a coated microparticle, a coated microsphere, a coated granule, a coated pellet, a tablet, a capsule or the like. Preferably, the pharmaceutical dosage form of the present invention is in the form of microcapsules which may be incorporated into an inert tablet matrix or into an inert capsule to enable their application and to assure exact dosing. The inert tablet matrix or the inert capsule must not influence the drug release.
The term 'microcapsule' as used herein refers to a spheroid particle, ranging from 1 µm to 3000 µm in size comprising a core and at least one coat. Microcapsules are incorporated into different dosage forms: suspensions, capsules or tablets, they may also be used as powders.

The term 'microsphere' as used herein, refers to a spheroid particle, ranging from 1 µm to 3000 µm comprising the active substance, a polysaccharide (forming a polysaccharide matrix) and one or more excipients.

A further embodiment of the present invention relates to the pharmaceutical dosage form where no outer coat is applied. Preferably, the pharmaceutical dosage form is in the form of microcapsules which comprises a core and an inner coat. Instead of applying the outer coat more microcapsules are embedded:
- either into a gastroresistant tablet matrix forming a tablet, or
- into an inert tablet matrix which is subsequently coated with a coat from a gastroresistant and/or acidoresistant polymer forming a tablet, or
- into a capsule from a gastroresistant and/or acidoresistant polymer, or
- into an inert capsule which is subsequently coated with a coat from a gastroresistant and/or acidoresistant polymer

Gastroresistant and/or acidoresistant polymers of said pharmaceutical dosage forms are soluble at pH above 5.5 (pH of intestinal fluid) and are selected from the group consisting of any acidoresistant polymer selected from the group consisting of derivatives of methacrylic acid copolymers (*e.g.* Eudragit^{®} L, Eudragit^{®} L-55, Eudragit^{®} S, Eudragit^{®} FS), HPMCP, hydroxyethylcellulose phthalate (HECP), cellulose acetate phthalate (CAP), polyvinyl acetyl phthalate (PAP), HPMC AS or combinations thereof.
Other substances may be added to modify drug release and/or to facilitate the preparation of tablets or capsules. For example, when HPMCP is used as gastroresistant polymer a mixture of PVAc and PVP (*e.g*. Kollidon^{®} SR) may be added to modify drug release.
Most preferably, the pharmaceutical dosage form of this embodiment of the present invention is a tablet comprising microcapsules (with a core and an inner coat) embedded into a tablet matrix which is preferably HPMCP combination with a mixture of PVAc and PVP (*e.g*. Kollidon^{®}SR).
In the pharmaceutical dosage form of this embodiment the proportion of the core is from about 10% to about 90% (w/w) to the total weight of the pharmaceutical dosage form. The proportion of the inner coat is from about 2% to about 50% by weight and the proportion of the tablet matrix from about 6% to about 90% to the total weight of the pharmaceutical dosage form. Preferably the proportion of the core in said dosage form is from about 10% to about 80% (w/w) to the total weight of the pharmaceutical dosage form. The proportion of the inner coat is from about 2% to about 30% (w/w) and the proportion of the tablet matrix is from about 6% to about 90% to the total weight of the pharmaceutical dosage form.

The pharmaceutical dosage forms of the present invention are administered to humans or animals in the amount from about 10 mg to about 1000 mg of LK 423 in a single dose or more divided doses.

The object of the present invention further relates to the processes for the preparation of the pharmaceutical dosage forms for controlled and/or targeted delivery of the active substance (e.g. LK 423).

The microcapsules of the present invention may be prepared by different methods. For the core preparation any method which enables the preparation of solid dispersions of the active substance in a polysaccharide, forming a polysaccharide matrix, in the form of beads, in particular the ionotropic gelation method based on rapid solidification of polysaccharide droplets in the solution of ions with which a polysaccharide forms insoluble salts, is suitable. Suitable methods are also spray drying, solvent extraction and evaporation and fluid bed methods. For some of these methods additional excipients may be needed.
Coating of the cores and microcapsules can be performed by employing the fluid bed technology or any other technology suitable for film coating. One of alternative methods is also solvent extraction or evaporation method.

The object of the present invention further relates to methods for controlled and/or targeted delivery of the active substance (e.g. LK 423) with the use or administration of any pharmaceutical dosage form of the present invention. Further, the object of present invention relates to the methods for controlled and/or targeted delivery of the active substance to all distal portions of gastrointestinal tract.

The object of the present invention further relates to the methods for the treatment of chronic inflammatory diseases in humans or animals by delivering of any pharmaceutical dosage form of the present invention. Chronic inflammatory diseases are selected from the group consisting of inflammatory bowel diseases in humans or animals, mainly colitis, nonspecific ulcerative colitis and/or Crohn's disease.
By using the method for the treatment of chronic inflammatory diseases the active substance is administered to humans or animals in the amount from about 10 mg to about 1000 mg daily in a single dose or more divided doses.
In addition to the methods of treatment, the object of the present invention is also use of the active substance for the preparation of any pharmaceutical dosage form of said invention for the treatment of chronic inflammatory disease in humans or animals. Chronic inflammatory disease is selected from the group consisting of inflammatory bowel diseases in humans or animals, mainly colitis, nonspecific ulcerative colitis and/or Crohn's disease.
By using the active substance for the preparation of any pharmaceutical dosage form of the present invention for the treatment of chronic inflammatory disease the active substance is administered to humans or animal in the amount from about 10 mg to about 1000 mg daily in a single dose or more divided doses.

### Example 1

### Preparation of microcapsules for the delivery of LK 423 to the rat colon

### Preparation of cores

The cores were prepared by the ionotropic gelation method, which is based on rapid solidification of polysaccharide solution droplets in a solution of ions with which polysaccharides form insoluble salts.
For the preparation of LMA pectin solutions (Genu pectin type LM-104 AS-Z, Hercules, Netherlands) pectin was being dissolved in demineralized water at least for 12 hours at room temperature. LK 423 and magnesium stearate were suspended in the pectin solution and the resulting dispersion was stirred for three hours on a magnetic stirrer. The dispersion was then poured manually by dropping into 0.25M solution of CaCl₂. The obtained cores were solidified in CaCl₂ for 30 minutes, filtered, washed with demineralised water and dried for 24 hours at room temperature and atmospheric pressure. Additionally, the cores were dried in Wurster chamber just prior to the coating process.
The composition of the dispersion for the preparation of cores is given in Table 1.

**Table 1: Composition of the dispersion for the preparation of cores**

| Ingredient | Amount |
|---|---|
| Pectin | 6.0 g |
| Demineralised water | 100 g |
| LK 423 | 6.0 g |
| Magnesium stearate | 2.7 g |

The dried cores were sieved in the further procedure and only a fraction of the particle size 1250-1600 µm was used for the preparation of microcapsules.

### Coating process

The coating process was performed by the fluid bed technology in the Apparatus Niro-Aeromatic STREA-1.

Compositions of the dispersions for core coating are described, in Table 2.

**Table 2: Composition of the dispersions for core coating.**

| Inner coat | | Outer coat | |
|---|---|---|---|
| Eudragit^{®} RS/RL 30 D (1:1) (30% w/w dispersion) | 48.33 g | Eudragit L 30 D-55 (30% w/w dispersion) | 53.0 g |
| Triethyl citrate | 3.63 g | Triethyl citrate | 1.6 g |
| Talc | 7.26 g | Talc | 8 g |
| Demineralized water | 40.78 g | Demineralized water | 37_{.}4 g |

*The weights of individual ingredients for the preparation of 100 g of the dispersion are given.*

For inner coating the following conditions were used:
- Inlet heated air flow: level 5,
- Inlet air temperature: 40°C,
- Spraying pressure: 2 bar,
- Coating suspension flow: 1.3 g/min.

The polymers in the inner coat represented 10% of the weight of uncoated cores.
The cores coated with the inner coat were cured for 24 hours at 40°C, and then the outer coat was applied under the following conditions:
- Inlet heated air flow: level 5
- Inlet air temperature: 40°C,
- Spraying pressure: 1.5 bar,
- Coating suspension flow: 1.9 g/min.

The polymer in the outer coat represented 30% of the weight of uncoated cores.

All tests were performed at least 24 hours after application of the outer coat. Meanwhile, the microcapsules were stored spread on the trays at room temperature.

As small quantities of the sample were available, the coating process was performed with a 1-2 g of the cores or cores with inner coat and a proper quantity of coloured placebo pellets which were clearly distinguishable by the colour were added up to 40 g. The microcapsules prepared in this way were from 1600-2000 µm in size. The content of LK 423 in the microcapsules was 14.5% (w/w).

### In vitro release test

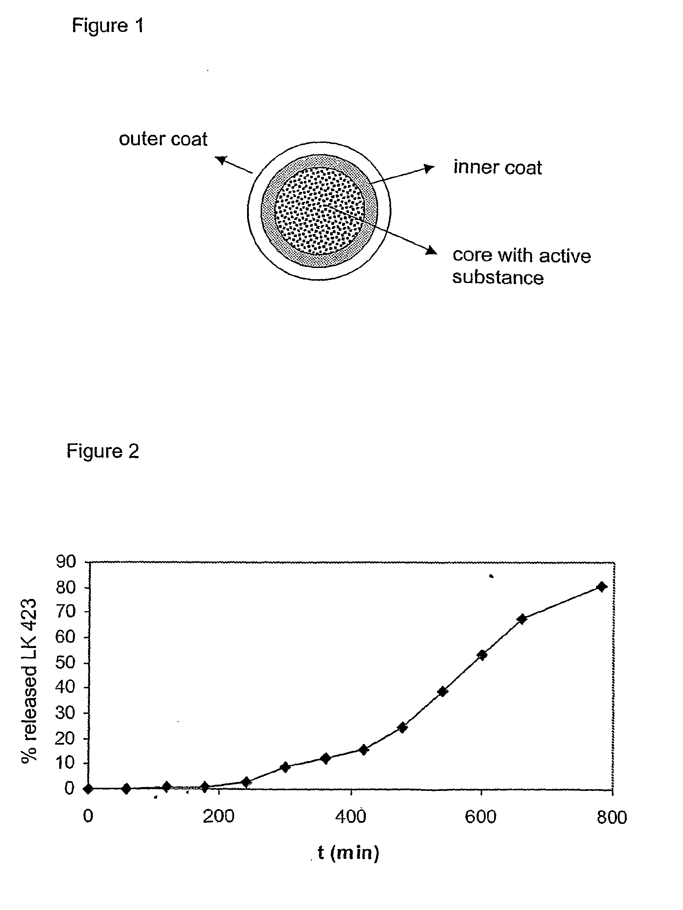

*In vitro* release test of LK 423 was carried out by using the above described microcapsules according to USP XXVI, in Apparatus 2, for 12 hours in different media in the following order: the first three hours in pH 3.3 HCl solution, three hours in pH 7.1 phosphate buffer, one hour in pH 7.6 phosphate buffer, and five hours in pH 7.1 phosphate buffer. These conditions should simulate the conditions in the gastrointestinal tract of the rat. The release profile of LK 423 is shown in Figure 2. The release profile of LK 423 shows that in first seven hours less than 20% of incorporated drug was released, followed by release of at least additional 50% of drug in next four hours.

### Example 2

Preparation of microcapsules for the delivery of LK 423 to the human colon.

The cores were prepared according to the method described in Example 1; cores of 1000-1600 µm in size were selected and then coated. The polymers in the inner coat (Table 2) represented 3% of the weight of uncoated cores. The polymer in the outer coat represented 30% of the weight of uncoated cores. The coating conditions are described in Example 1.
The microcapsules were 1250-2000 µm in size. The content of the active substance was 10.8% (w/w).

### In vitro release test

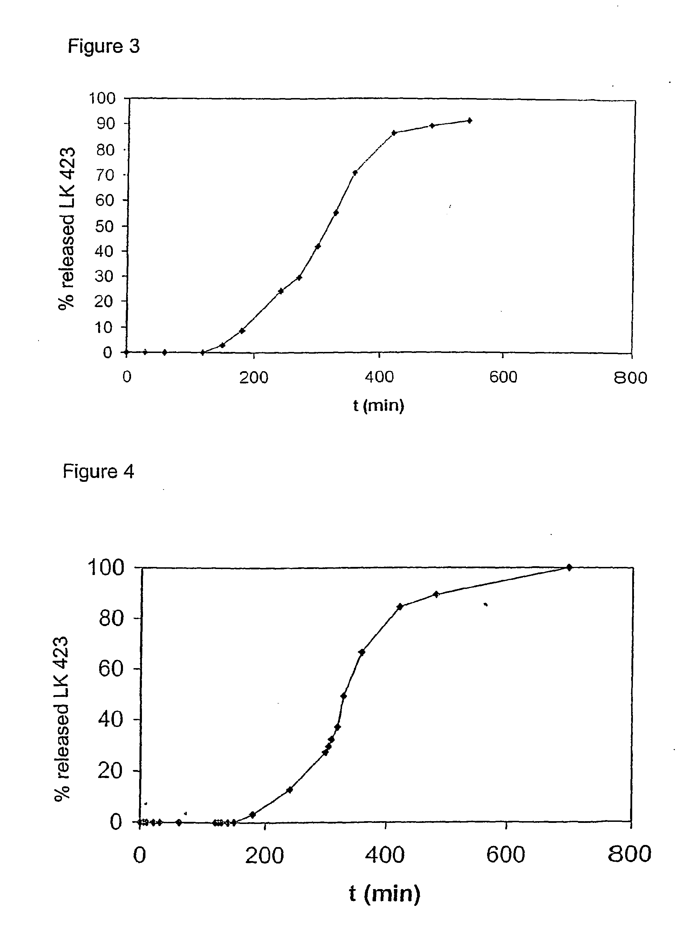

*In vitro* release test was carried out by using the above described microcapsules according to USP XXVI, in Apparatus 2, for two hours in pH 1.2 HCl solution, followed by 2-hour release in a pH 6.8 phosphate buffer, thereafter the microcapsules were placed in a pH 7.5 phosphate buffer for 1 hour, and finally in a pH 6.0 phosphate buffer for 4 hours. These conditions should simulate the conditions in human gastrointestinal tract. The release profile of LK 423 is shown in Figure 3.

The release profile of LK 423 shows that less than 15% of drug was released in first three hours of the test and at least additional 60% in next four hours.

### Example 3

### Preparation of microcapsules for the delivery of LK 423

The cores were prepared according to the method described in Example 1.
For the preparation of LMA pectin solutions (Genu pectin type LM-104 AS-Z, Hercules, Netherlands) pectin was being dissolved in demineralized water at least for 12 hours at room temperature. LK 423 and Aerosil 200 (Degussa) were suspended in the pectin solution and the resulting dispersion was stirred for 1 to 2 hours on a magnetic stirrer. The dispersion was then poured by dropping into ice-cold 0.272 M solution of CaCl₂ with aid of infusion pump. For the amount of approximately 2.4 g of dispersion 100 mL of CaCl₂ was used. The obtained cores were solidified in CaCl₂ for 5 minutes, filtered, washed with demineralised water and dried for 24 hours at room temperature and atmospheric pressure. Additionally, the cores were dried in Wurster chamber just prior to the coating process. The amounts of substances used are shown in Table 1.

**Table 3: Composition of the dispersion for the preparation of cores.**

| Ingredient | Amount |
|---|---|
| Pectin | 3.5 g |
| Demineralised water | 100 g |
| LK 423 | 3.5 g |
| Aerosil 200 | 5.512 g |

The dried cores were sieved in the further procedure and only a fraction of the particle size 1600-2000 µm was used for preparation of microcapsules.

The coating conditions are described in Example 1, with the exception that the ratio of polymers in the inner coat Eudragit RS : Eudragit RL was 7 : 3. The polymers in the inner coat represented 15% of the weight of uncoated cores. The polymers in the outer coat represented 30% of the weight of uncoated cores

The microcapsules were 1600-2000 µm in size. The content of the active substance was 14,11%.

### In vitro release test

*In vitro* release test of LK 423 was carried out by using the above described microcapsules according to USP XXVI, in Apparatus 2, in different media in the following order: the first two hours in pH 1,2 HCl solution containing 2g/L NaCl and to the end in pH 6.8 phosphate buffer. The release profile of LK 423 shows that in first five hours less than 25% of incorporated drug was released, followed by release of at least additionally 55% of drug in next five hours.

### Example 4

### Preparation of tablets for the delivery of LK 423 to the human colon

The cores comprised a solid dispersion of LK 423 in the calcium pectinate (forming a calcium pectinate matrix) coated with Eudragit^{®} RS polymer. The obtained microcapsules were embedded into the tablet matrix which was a combination of HPMCP and Kollidon^{®} SR. The microcapsules were entirely coated with the combination of said two polymers.

### Preparation of cores

The cores were prepared by the ionotropic gelation method. The process was conducted as described in Example 1 with the exception that no magnesium stearate was used. The ratio of pectin LMA (GENU pectin LMA, Hercules) to LK 423 in the initial dispersion was 1 : 3 (w/w). The amount of water per g of pectin in initial dispesion was between 25 and 30g.

### Coating process

The inner coat was applied by the solvent evaporation method in the system acetone / liquid paraffin with approximate ratio 6mL / 80mL. 0.5 g of cores were suspended in acetone in which Eudragit^{®} RS (3.0 g) had been dissolved and magnesium stearate (0.2 g) suspended. Acetone was evaporated during stirring at 40°C and the resulting microcapsules were dried at room temperature and reduced pressure overnight. No outer coat was applied. The fraction 1250 - 1600 µm was used in tabletting process. The content of LK 423 in the microcapsules was 62% (w/w).

### Tabletting process

The tablets were compressed on a tabletting machine. The microcapsules were placed onto a layer of a physical mixture of HPMCP (HP-55, Shin-Etsu) and Kollidon^{®} SR (BASF) and onto them again a layer of HPMCP and Kollidon^{®} SR mixture. The mixture of polymers was also applied on both lateral surfaces of the tablets in order to prevent direct contact of microcapsules with the environment. For one tablet 150 mg (2 x 75 mg) of HPMCP, 20 mg (2 x 10 mg) of Kollidon^{®} SR and 32 mg of microcapsules were used. The content of the LK 423 was 20 mg per tablet, that is, 9.9% (w/w).

### In vitro release test

*In vitro* release test of LK 423 was carried out by using the above described tablets according to USP XXVI, in Apparatus 2, in different media in the following order: the first two hours in pH 1,2 HCl solution containing 2g/L NaCl, three hours in pH 6.8 phosphate buffer, and to the end in pH 6.0 Sorensen's phosphate buffer with added pectinase (Pectinec Ultra SP-L, Novo Nordisk). These conditions should simulate the conditions in human gastrointestinal tract. The release profile of LK 423 is shown in Figure 4. The release profile of LK 423 shows that in first three hours less than 15% of incorporated drug was released, followed by release of at least additionally 60% of drug in next four hours.

### Example 5

Demonstration of antiulcerative action *in vivo* of the microcapsules with LK 423

Ulcerative changes of ileum, caecum and colon in rats were developed in five days by drinking water containing 2% of sodium dextran sulphate (DSS).
DSS is the compound which after oral administration in an aqueous solution induces inflammatory and ulcerative changes in the mucosa of ileum, caecum and colon in animals.
Two control groups and two experimental groups (A and B) of rats were formed, each group had the same number of male and female rats. The animals were randomly assigned to the individual groups. Until 7 p.m. of day 5 the animals had a free access to food, thereafter feeding was allowed only between 12 a.m. and 6 p.m.; meantime, the animals had no access to food.

From experiment day 6 onward the animals were drinking water with 1% DSS. The animals in Group A received the pure compound LK 423 in a dose of 75 mg/kg. The pure compound LK 423 was prepared as suspension in 1% gelatin (Gelatin GE0020, Scharlau) and was given orally via the tube in a volume of 1 ml.
In Group B the animals received the microcapsules comprising LK 423 in a dose of 75 mg/kg (as prepared in Example 1). The microcapsules comprising LK 423 were given orally via the tube dispersed in 1.5 to 2 ml 1 % of gelatin.

At baseline the rats had body weight from 220 to 250 g. During the experiment the survived animal did not significantly change body weight in any of the groups. At baseline and on days 6 and 12 the body weight was recorded. For determination of the hematology parameters 1.5 ml blood samples were taken from the periorbital sinus at baseline and on day 5 of the experiment and from the abdominal aorta on day 12. The erythrocyte count and hemoglobin and hematocrit values were performed in the clinical laboratory of the Institute for Health Protection of Ruminants, Faculty of Veterinary Science. On day 1 of the experiment the values for the erythrocytes ranged from 6.93 to 7.87 x 10x E12/l hemoglobin 139 to 149.7 g/l and hematocrit 41.81 to 42.5% in the individual groups. Throughout the experiment survival of the animals, diarrhea and occurrence of blood in the feces or the intensity of bleeding were also observed. On day 12 the animals were sacrificed under deep ether anesthesia and post-mortem examination was performed. Macroscopic changes in the ileum, caecum and in the colon and the intensity of bleeding were observed.

At autopsy fragments of the intestine were taken from the animals and fixed in 10% formaldehyde; paraffin tissue sections were prepared and stained with hematoxylin and eosin. Pathohistological diagnosis was then performed.

DSS-induced changes included coagulation necrosis of the intestinal mucosa. In all groups (both control groups, group A and group B) (Table 3) the tissue damage and a reaction of the surrounding tissue to the damage (level of regeneration) as well as accompanying parameters of the scope of bleeding in the mucosa, hypertrophy of the mucosa with glandular hyperplasia and the degree of epithelialization of the damage surface were assessed. The criteria were as follows:
The scope of acute bleeding with respect to the tissue and to the diameter of the field of view at 40-fold magnification (4 mm):
- No bleeding - 0
- Small bleeding in the propria and submucosa, with a diameter less than half of the field of view - 1
- Moderate bleeding in the propria and submucosa, with a diameter to one half of the field of view - 2
- Very extensive bleeding in the propria and submucosa with a diameter greater than half of the field of view - 3
- Very extensive bleeding in the propria, submucosa, around the vessels in the tunica muscularis, tunica serosa, and in the supporting tissue - 4
   Regressive process - damage with a defense reaction of the tissue:

■ No cellular response (no reaction) or the regressive process was no longer active -0
■ Moderate cellular infiltration (monocytes, granulocytes, macrophage, histocytes) with karyorrhesis and pyknosis in the mucosa and the submucosa - 1
■ Severe diffuse cellular infiltration (monocytes, granulocytes, macrophage, histocytes) with karyorrhesis and pyknosis in the mucosa and the submucos - 2
■ Cellular infiltration (monocytes, granulocytes, macrophage, histocytes) in the demarcation line around the injury - 3
   Regenerative process / the tissue organisation:

■ No tissue response - 0
■ Initial phase (tissue infiltration with fibrin, phagocytes, monocytes, reticular mesenchymal cells, endothelial cells) -1
■ Granulation tissue (fibroblasts, histiocytes, lymphocytes, monocytes) -2
■ Mature granulation tissue with newly formed basic cells of the glandular epithelium, lymphocytes, collagen fibres -3
■ Damage is restored - 4
   Hypertrophy of the mucosa and hyperplasia of the glands and goblet cells:
■ None - 0
■ Moderate around the damage - 1
■ Regenerative - 2
■ Excessive - 3
   Epithelialization of damages:
■ None - 0
■ Initial - 1
■ Regenerative - 2
■ Excessive - 3

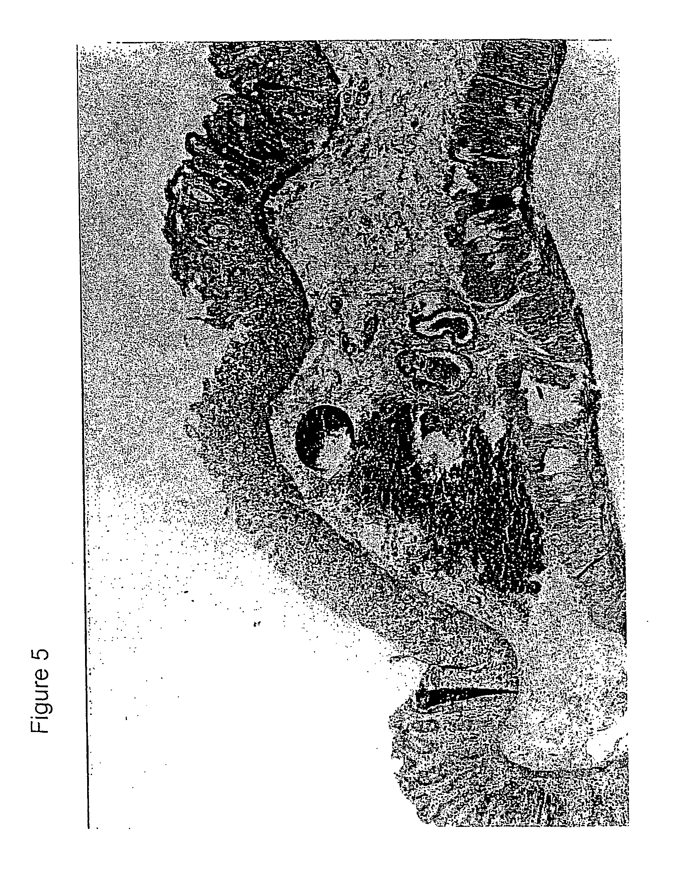

The assessment of the level of damage and restoration and the scope of bleeding in the mucosa is presented for the control groups in Table 3. In both control groups the induced tissue necrosis was accompanied by inflammatory cell infiltration. In fresh damages the cell infiltration was diffused; in the cells themselves karyorrhesis was visible. In older damages the cell infiltration was organized into the demarcation line which marked off the damaged part of the tissue from *de novo* formed granulation tissue. Hypertrophy of the mucosa was visible at the margins of the damage; epithelialization on the surface of the damage was not initiated yet (Figure 5). In the control groups necrosis of the tissue and confining the damage was most manifested. Regeneration processes were not visible.

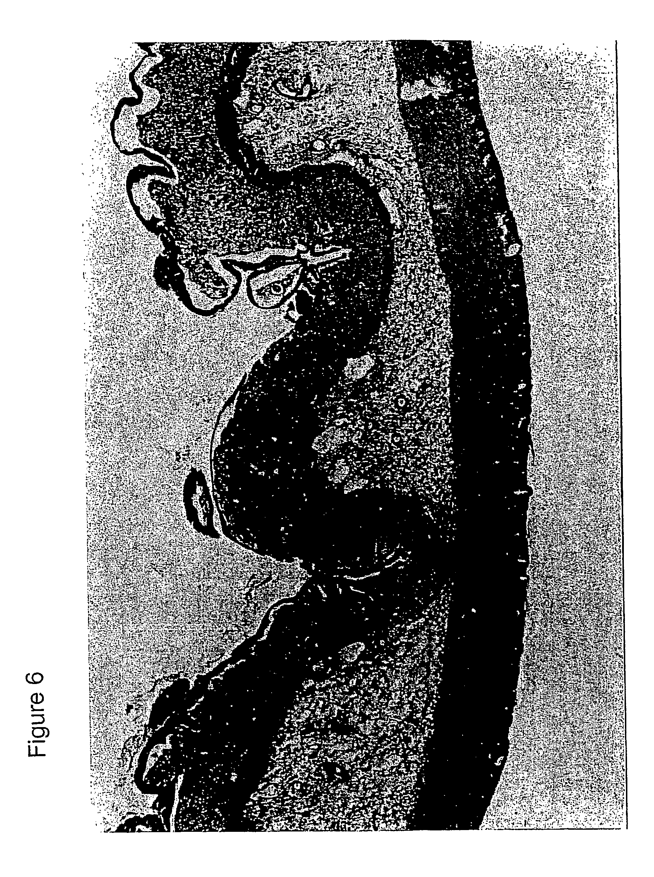

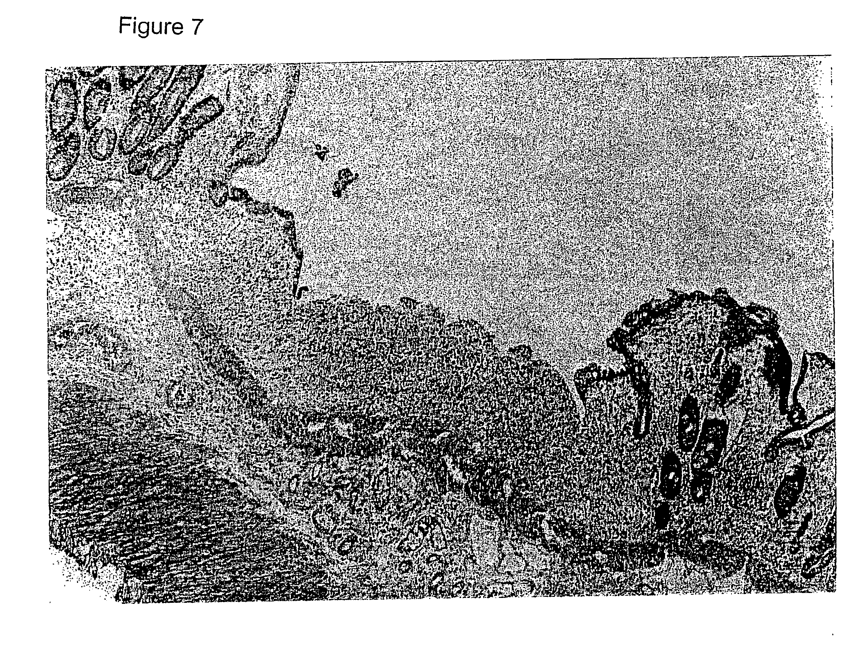

In the group A, DSS-induced tissue necrosis was accompanied by cellular infiltration, growth of granulation connective tissue and at the same time as well pronounced regenerative processes. The process of confining the damage was still active. The process of functional restoration of the mucosa manifested in excessive growth of the glandular and superficial epithelium. Despite strong hyperplasia of the surrounding tissue, sites renewed with mature connective tissue only were observed (Table 3, Figure 6). The regenerative process / damage level ratio was 1.35 : 1 in this group.
In the group B, DSS-induced tissue necrosis was accompanied by moderate cellular infiltration. After treatment the damaged tissue was replaced by *de novo* formed immature connective tissue with all tissue components of the intestinal mucosa (superficial and glandular epithelium, lymph follicles, and vessels). The regeneration processes were manifested at the tissue level (Table 3, Figure 7). The regenerative process / damage level ratio was 3.22 : 1.

The presence of blood in the feces, the percent of animals with bleeding in the individual bowel segments and the level of bleeding in the mucosa and the submucosa are also shown in Table 3. Decrease in haematological parameters from baseline to end of the experiment and the mortality in individual groups are presented.

**Table 3: Effect of pure compound LK 423 (group A) and microcapsules comprising LK 423 (group B) on ulcerative changes of the ileum, caecum and colon**

| Group | Control | Group A (Pure compound) | Control | Group B (Microcapsules) |
|---|---|---|---|---|
| Dose of the active substance | 0 | 75 mg/kg | 0 | 75 mg/kg |
| Number of animals | 6 | 6 | 10 | 10 |
| | | | | |
| Blood in the feces * | ++ to +++ | + | ++ to +++ | + |
| | | | | |
| Level of bleeding in the mucosa # | 2.0 | 0.6 | 2.0 | 0.2 |
| | | | | |

| Percent of bleeding animals | | | | |
|---|---|---|---|---|
| Ileum | 10 | 17 | 30 | 0 |
| Caecum | 50 | 17 | 40 | 0 |
| Colon | 50 | 17 | 70 | 0 |
| | | | | |

| Difference of hematology values from day 0 to 12 | | | | |
|---|---|---|---|---|
| Erythrocytes x 10¹²/l (x 10 x E12/l) | 2.20 | 1.25 | 1.78 | 1.02 |
| Hemoglobin g/l | 41.6 | 17.2 | 23.7 | 2.3 |
| Hematocrit % | 12.9 | 6.74 | 7.6 | 4.0 |
| | | | | |

| Mortality | | | | |
|---|---|---|---|---|
| Mortality in % | 33 | 33 | 50 | 0 |
| | | | | |

| Cellular process in sacrificed animals# | | | | |
|---|---|---|---|---|
| Level of damage | 2.5 | 1.75 | 2.2 | 0.9 |
| Level of regeneration | 0 | 2.37 | 0 | 2.90 |
| Hypertrophy of the mucosa | 0.87 | 2.75 | 1.87 | 1.80 |
| Epithelialization | 0 | 2.37 | 0.37 | 1.20 |

| | | | | |
|---|---|---|---|---|
| * + blood in traces, ++ moderate bleeding, +++ strong bleeding # described from 0 to 3; for individual level see the example | | | | |

From Table 3 and Figures 5 - 7 it can be concluded that incorporation of LK 423 into the microcapsules essentially improves the therapeutic oral action of LK 423. Improvement is manifested by reduced bleeding in different portions of the bowel, in higher survival of animals and more rapid and more physiological renewal of the intestinal mucosa. More rapid tissue renewal in the group B (microcapsules with LK 423) contributes to minor changes in hematological parameters compared to the control groups or the group A (with the pure compound LK 423).

In the animals receiving the pure compound LK 423 or the microcapsules comprising LK 423 a more rapid regeneration of damages of the intestinal mucosa was observed than in both control groups. In the animals receiving the pure compound LK 423 regeneration did not take place uniformly in all tissue segments. The intestinal epithelial layer which was covering the propria in which an inflammatory process was still active was rapidly restored (renewed). The glandular epithelium surrounding the damaged tissue and independent of the damage manifested the signs of hyperplasia which exceeded the level attained in the group receiving the microcapsules comprising LK 423 (group B) of the present invention. In the group receiving the pure compound LK 423 (group A) a strong reaction of lymph follicles (hyperplasia) in the mucosa and deeper in the submucosa was observed. In the animals receiving microcapsules comprising LK 423 of the present invention regeneration of the mucosa damages was more uniform and harmonized resulting in the physiological regeneration of the intestinal mucosa, the inflammatory process in the mucosa was confined and eliminated. Covering the areas of the granulation tissue by the intestinal epithelial layer was initiated from the margins of the damage. Hyperplasia of the epithelium along the margin of the damage was within the physiological limits. Regeneration of the induced damages of the intestinal mucosa in the animals receiving the microcapsules of the present invention was more physiological than in the group receiving the pure compound LK 423 and it involved all tissue components of the mucosa uniformly and without excessive growth.

The results show that the microcapsules of the present invention, where the active substance LK 423 is incorporated into the core of calcium pectinate coated with two coats, prevented release of LK 423 in the stomach due to outer coat. However, the inner coat provided sustained, delayed and controlled release of LK 423 along the small intestine and initiated the release of LK 423 in the distal portion of the small intestine and preferably in the large intestine. Upon entering the large intestine, where the intestinal bacteria degrade polysaccharide cores, the release of LK 423 was accelerated and LK 423 was then rapidly released.
The structure of a controlled release pharmaceutical dosage form of the present invention afforded delivery of the suitable concentrations of the active substance LK 423 to the diseased sites with convenient kinetics. These features provided an optimal action of the pharmaceutical dosage form of the present invention.

## Claims

1. A gastroresistant pharmaceutical dosage form comprising a core which comprises the active substance N-(2-(2-phthalimidoethoxy)-acetyl)-L-alanyl-D-glutamic acid and a polysaccharide, and an inner coat which comprises a polymer, suitable for controlled and / or targeted delivery of the said active substance to the distal portions of the gastrointestinal tract of humans and animals.

2. The gastroresistant pharmaceutical dosage form according to claim 1 wherein the distal portions of the gastrointestinal tract are ileum, ceacum and colon.

3. The gastroresistant pharmaceutical dosage form according claim 1 wherein the dosage form is administered to humans or animals in the amount from about 10 mg to about 1000 mg of the active substance according to claim 1 in a single dose or more divided doses.

4. The gastroresistant pharmaceutical dosage form according to claim 1 wherein the polysaccharide is selected from the group consisting of pectin or alginate, either in the form of acid or in the form of metal salt, galactomannans, covalently crosslinked dextran, amylose, xanthans, carrageenan and starch or combinations of the said polysaccharides or their salts with the same specific degradability.

5. The gastroresistant pharmaceutical dosage form according to claim 4 wherein the polysaccharide is selected from the group consisting of pectin and calcium pectinate.

6. The gastroresistant pharmaceutical dosage form according to claim 1 wherein the core is a solid dispersion of the active substance in the calcium pectinate, forming a calcium pectinate matrix.

7. The gastroresistant pharmaceutical dosage form according to claim 1 wherein the core further comprises a glidant selected from the group consisting of magnesium stearate, calcium stearate and aerosil.

8. The gastroresistant pharmaceutical dosage form according to claim 1 wherein the inner coat prevents the release of the active substance in the proximal portions of the small intestine.

9. The gastroresistant pharmaceutical dosage form according to claim 1 wherein the inner coat comprises a polymer selected from the group consisting of methacrylate ester compolymers, mixture of polyvinyl acetate and polyvinylpyrrolidone and/or combinations thereof.

10. The gastroresistant pharmaceutical dosage form according to claim 9 wherein the selected combination of polymers is combination of copolymers of acrylic and methacrylic acid esters with a low content of quartenary ammonium groups.

11. The gastroresistant pharmaceutical dosage form according to claim 1 wherein the dosage form further comprises an outer coat which is insoluble in an acidic environment at pH below 5 and prevents release of the active substance in the acidic medium of the stomach.

12. The pharmaceutical dosage form according to claim 11 wherein the outer coat comprises an acidoresistant polymer selected from the group consisting of derivatives of methacrylic acid copolymer, hydroxypropylmethyl cellulose phthalate, hydroxyethylcellulose phthalate, cellulose acetate phthalate, polyvinyl acetyl phthalate, hydroxypropylmethylcellulose acetate succinate or combinations thereof.

13. The gastroresistant pharmaceutical dosage form according to claim 12 wherein the acidoresistant polymer is an anionic copolymer based on methacrylic acid and ethyl acrylate.

14. The gastroresistant pharmaceutical dosage forms according to claims 1 and 11 wherein the coats further comprises a glidant selected from the group consisting of talc, kaolin and glycerol monostearate.

15. The gastroresistant pharmaceutical dosage form according to claim 14 wherein the glidant is talc.

16. The gastroresistant pharmaceutical dosage forms according the claims 1 and 11 wherein the coats further comprise a plasticizer selected from the group consisting of triethyl citrate, tributyl citrate, acetyltriethyl citrate, acetyltributyl citrate, diethyl phthalate, dibutyl phthalate, dibutyl sebacate, glyceryl triacetate, triacetin, polyethylene glycol 6000 and polyoxyethylene (20) sorbitan monooleate.

17. The gastroresistant pharmaceutical dosage form according to claim 16 wherein the plasticizer is triethyl citrate.

18. A gastroresistant pharmaceutical dosage form comprising:
• a core - a calcium pectinate matrix in which the active substance N-(2-(2-phthalimidoethoxy)-acetyl)-L-alanyl-D-glutamic acid is dispersed, comprising magnesium stearate, and
• inner coat - comprising polymers Eudragit RS and Eudragit RL, talc and triethyl citrate, and
• outer coat - comprising polymer Eudragit L-55, talc and triethyl citrate.

19. The gastroresistant pharmaceutical dosage form according to claim 1 wherein the dosage form is in a form of a microcapsule which is embedded
• either into a gastroresistant tablet matrix forming a tablet, or
• into an inert tablet matrix which is subsequently coated with a coat from a gastroresistant and/or acidoresistant polymer forming a tablet, or
• into a capsule from a gastroresistant and/or acidoresistant polymer, or
• into an inert capsule which is subsequently coated with a coat from a gastroresistant and/or acidoresistant polymer.

20. The gastroresistant pharmaceutical dosage form according to claim 19 wherein the dosage form is a tablet comprising microcapsules embedded into a gastroresistant tablet matrix.

21. The gastroresistant pharmaceutical dosage form according to claim 20 where the tablet matrix is hydroxypropylmethyl cellulose phthalate combination with a mixture of polyvinyl acetate and polyvinylpyrrolidone.

22. The gastroresistant pharmaceutical dosage form according to claim 19 wherein the gastroresistant and/or acidoresistant polymer is selected from the group consisting of derivatives of methacrylic acid copolymer, hydroxypropylmethyl cellulose phthalate, hydroxyethyl cellulose phthalate, cellulose acetate phthalate, polyvinyl acetyl phthalate, hydroxypropylmethylcellulose acetate succinate or combinations thereof.

## Patentansprüche

1. Magensaftresistente pharmazeutische Dosierform, die einen Kern, der die aktive Substanz N-(2-(2-Phthalimidoethoxy)acetyl)-L-alanyl-D-glutaminsäure und ein Polysaccharid umfasst, und eine innere Beschichtung umfasst, welche ein Polymer umfasst, das zur kontrollierten und/oder gezielten Abgabe der aktiven Substanz an die distalen Bereiche des Gastrointestinaltrakts von Menschen und Tieren geeignet ist.

2. Magensaftresistente pharmazeutische Dosierform nach Anspruch 1, bei der die distalen Bereiche des Gastrointestinaltrakts Ileum, Zäkum und Kolon sind.

3. Magensaftresistente pharmazeutische Dosierform nach Anspruch 1, bei der die Dosierform Menschen oder Tieren in der Menge von etwa 10 mg bis etwa 1000 mg der aktiven Substanz gemäß Anspruch 1 in einer Einzeldosis oder mehreren unterteilten Dosen verabreicht wird.

4. Magensaftresistente pharmazeutische Dosierform nach Anspruch 1, bei der das Polysaccharid ausgewählt ist aus der Gruppe bestehend aus Pektin oder Alginat in Form der Säure oder eines Metallsalzes, Galactomannanen, kovalent vernetztem Dextran, Amylose, Xanthanen, Carrageen und Stärke oder Kombinationen dieser Polysaccharide oder ihrer Salze mit derselben spezifischen Abbaubarkeit.

5. Magensaftresistente pharmazeutische Dosierform nach Anspruch 4, bei der das Polysaccharid ausgewählt ist aus der Gruppe bestehend aus Pektin und Calciumpektinat.

6. Magensaftresistente pharmazeutische Dosierform nach Anspruch 1, bei der der Kern eine feste Dispersion der aktiven Substanz in dem Calciumpektinat ist, unter Bildung einer Calciumpektinatmatrix.

7. Magensaftresistente pharmazeutische Dosierform nach Anspruch 1, bei der der Kern ferner ein Fließregulierungsmittel ausgewählt aus der Gruppe bestehend aus Magnesiumstearat, Calciumstearat und Aerosil umfasst.

8. Magensaftresistente pharmazeutische Dosierform nach Anspruch 1, bei der die innere Beschichtung die Freisetzung der aktiven Substanz in den proximalen Bereichen des Dünndarms verhindert.

9. Magensaftresistente pharmazeutische Dosierform nach Anspruch 1, bei der die innere Beschichtung ein Polymer ausgewählt aus der Gruppe bestehend aus Methacrylatestercopolymeren, Mischung von Polyvinylacetat und Polyvinylpyrrolidon und/oder Kombinationen davon umfasst.

10. Magensaftresistente pharmazeutische Dosierform nach Anspruch 9, bei der die gewählte Kombination von Polymeren eine Kombination von Copolymeren von Acryl- und Methacrylsäureestern mit einem niedrigen Gehalt an quaternären Ammoniumgruppen ist.

11. Magensaftresistente pharmazeutische Dosierform nach Anspruch 1, bei der die Dosierform ferner eine äußere Beschichtung umfasst, die in einer sauren Umgebung bei einem pH-Wert unter 5 unlöslich ist und die Freisetzung der aktiven Substanz im sauren Medium des Magens verhindert.

12. Pharmazeutische Dosierform nach Anspruch 11, bei der die äußere Beschichtung ein säurebeständiges Polymer ausgewählt aus der Gruppe bestehend aus Derivaten von Methacrylsäurecopolymer, Hydroxypropylmethylcellulosephthalat, Hydroxyethylcellulosephthalat, Celluloseacetatphthalat, Polyvinylacetylphthalat, Hydroxypropylmethylcelluloseacetatsuccinat oder Kombinationen davon umfasst.

13. Magensaftresistente pharmazeutische Dosierform nach Anspruch 12, bei der das säurebeständige Polymer ein anionisches Copolymer ist, das auf Methacrylsäure und Ethylacrylat basiert.

14. Magensaftresistente pharmazeutische Dosierform nach den Ansprüchen 1 und 11, bei denen die Beschichtung ferner ein Fließregulierungsmittel ausgewählt aus der Gruppe bestehend aus Talkum, Kaolin und Glycerinmonostearat umfasst.

15. Magensaftresistente pharmazeutische Dosierform nach Anspruch 14, bei der das Fließregulierungsmittel Talkum ist.

16. Magensaftresistente pharmazeutische Dosierform nach Ansprüchen 1 und 11, bei denen die Beschichtung ferner einen Weichmacher ausgewählt aus der Gruppe bestehend aus Triethylcitrat, Tributylcitrat, Acetyltriethylcitrat, Acetyltributylcitrat, Diethylphthalat, Dibutylphthalat, Dibutylsebacat, Glyceryltriacetat, Triacetin, Polyethylenglykol 6000 und Polyoxyethylen(20)sorbitanmonooleat umfasst.

17. Magensaftresistente pharmazeutische Dosierform nach Anspruch 16, bei der der Weichmacher Triethylcitrat ist.

18. Magensaftresistente pharmazeutische Dosierform, umfassend:
• einen Kern-eine Magnesiumstearat umfassende Calciumpektinatmatrix, in der die aktive Substanz N-(2-(2-Phthalimidoethoxy)acetyl)-L-alanyl-D-glutaminsäure dispergiert ist, und
• innere Beschichtung -welche die Polymere Eudragit RS und Eudragit RL, Talkum und Triethylcitrat umfasst, und
• äußere Beschichtung welche das Polymer Eudragit L-55, Talkum und Triethylcitrat umfasst.

19. Magensaftresistente pharmazeutische Dosierform nach Anspruch 1, die in Form einer Mikrokapsel vorliegt, die eingebettet ist in
• eine magensaftresistente Tablettenmatrix, die eine Tablette bildet, oder
• eine inerte Tablettenmatrix, die nachfolgend mit einer Beschichtung aus einem magensaftresistenten und/oder säurebeständigen Polymer beschichtet wird, um eine Tablette zu bilden, oder
• eine Kapsel aus einem magensaftresistenten und/oder säurebeständigen Polymer, oder
• eine inerte Kapsel, die nachfolgend mit einer Beschichtung aus einem magensaftresistenten und/oder säurebeständigen Polymer beschichtet wird.

20. Magensaftresistente pharmazeutische Dosierform nach Anspruch 19, die eine Tablette ist, welche in eine magensaftresistente Tablettenmatrix eingebettete Mikrokapseln umfasst.

21. Magensaftresistente pharmazeutische Dosierform nach Anspruch 20, bei der die Tablettenmatrix Hydroxypropylmethylcellulosephthalat in Kombination mit einer Mischung aus Polyvinylacetat und Polyvinylpyrrolidon ist.

22. Magensaftresistente pharmazeutische Dosierform nach Anspruch 19, bei der das magensaftresistente und/oder säurebeständige Polymer ausgewählt ist aus der Gruppe bestehend aus Derivaten von Methacrylsäurecopolymer, Hydroxypropylmethylcellulosephthalat, Hydroxyethylcellulosephthalat, Celluloseacetatphthalat, Polyvinylacetylphthalat, Hydroxypropylmethylcelluloseacetatsuccinat oder Kombinationen davon.

## Revendications

1. La forme galénique pharmaceutique gastrorésistante avec un noyau qui comprend la substance active d'acide N-(2-(2-phthalimidoethoxy)-acetyl)-L-alanyl-D-glutamique et un polysaccharide, ainsi qu'une couche intérieure comprenant un polymère adapté à une administration contrôlée et / ou ciblée de ladite substance active aux parties distales du tractus gastro-intestinal des humains et des animaux.

2. La forme galénique pharmaceutique gastrorésistante, selon la revendication 1, dans laquelle les parties distales du tractus gastro-intestinal sont l'iléon, le cecum et le côlon.

3. La forme galénique pharmaceutique gastrorésistante, selon la revendication 1, dans laquelle la forme dosifiée est administrée aux humains ou aux animaux en une quantité d'environ 10 mg à 1000 mg de la substance active, selon la revendication 1, en une dose unique ou en plusieurs doses fractionnées.

4. La forme galénique pharmaceutique gastrorésistante, selon la revendication 1, dans laquelle le polysaccharide est choisi à partir du groupe composé de pectine ou d'alginate, soit sous forme d'acide ou sous forme de sel de métal, de galactomannanes, de dextran réticulé en covalence, d'amylose, de xanthanes, de carraghénane et d'amidon ou des combinaisons desdites polysaccharides ou leurs sels avec la même dégradabilité spécifique.

5. La forme galénique pharmaceutique gastrorésistante, selon la revendication 4, dans laquelle le polysaccharide est choisi à partir du groupe composé de pectine et de pectinate de calcium.

6. La forme galénique pharmaceutique gastrorésistante, selon la revendication 1, dans laquelle le noyau est une dispersion solide de la substance active dans le pectinate de calcium formant une matrice de pectinate de calcium.

7. La forme galénique pharmaceutique gastrorésistante, selon la revendication 1, dans laquelle le noyau comprend en outre un agent de glissement choisi à partir du groupe composé de stéarate de magnésium, de stéarate de calcium et d'aérosil

8. La forme galénique pharmaceutique gastrorésistante, selon la revendication 1, dans laquelle la couche intérieure prévient la libération de la substance active dans les segments proximaux de l'intestin grêle.

9. La forme galénique pharmaceutique gastrorésistante, selon la revendication 1, dans laquelle la couche intérieure comprend un polymère choisi à partir du groupe composé de copolymères d'ester méthacrylate, un mélange d'acétate de polyvinyle et de polyvinylpyrrolidone et / ou de leurs combinaisons.

10. La forme galénique pharmaceutique gastrorésistante, selon la revendication 9, dans laquelle la combinaison de polymères choisie est une combinaison de copolymères d'esters des acides acryliques et méthacryliques avec une faible teneur des groupes ammonium quaternaire.

11. La forme galénique pharmaceutique gastrorésistante, selon la revendication 1, dans laquelle la forme dosifiée comprend en outre une couche extérieure qui est insoluble dans un environnement acide avec un pH inférieur à 5 et empêche la libération de la substance active dans l'environnement acide de l'estomac.

12. La forme galénique pharmaceutique gastrorésistante, selon la revendication 11, dans laquelle la couche extérieure comprend un polymère acidorésistant choisi à partir du groupe composé des dérivés de copolymère d'acide méthacrylique, de phtalate d'hydroxypropylméthylcellulose, de phtalate d'hydroxyéthylcellulose, de phtalate acétylcellulose, de phtalate de polyvinyle acétyle, d'acéto-succinate d'hydroxypropylméthylcellulose ou de leurs combinaisons.

13. La forme galénique pharmaceutique gastrorésistante, selon la revendication 12, dans laquelle le polymère acidorésistant est un copolymère anionique à base d'acide méthacrylique et d'acrylate d'éthyle.

14. La forme galénique pharmaceutique gastrorésistante, selon les revendications 1 et 11, dans lesquelles les couches comprennent en outre un agent de glissement choisi à partir du groupe composé de talc, de kaolin et de monostéarate de glycérol.

15. La forme galénique pharmaceutique gastrorésistante, selon la revendication 14, dans laquelle l'agent de glissement est le talc.

16. La forme galénique pharmaceutique gastrorésistante, selon les revendications 2 et 11, dans lesquelles les couches comprennent en outre un plastifiant choisi à partir du groupe composé de citrate d'éthyle, de citrate de tributyle, de citrate acétyltriéthyle, de citrate acétyltributylique, de phtalate de diéthyle, de phtalate de dibutyle, de sébacate de butyle, de triacétate du glycérol, de triacétine, de polyéthylène glycol 6000 et de polyoxyéthyléné (20) mono-oléate de sorbitane

17. La forme galénique pharmaceutique gastrorésistante, selon la revendication 16, dans laquelle le plastifiant est le citrate d'éthyle.

18. Une forme galénique pharmaceutique gastrorésistante comprenant :
• un noyau - une matrice de pectinate de calcium dans laquelle la substance active d'acide N-(2-(2-phthalimidoethoxy)-acetyl)-L-alanyl-D-glutamique est dispersée, et comprenant un stéréate de magnésium ; et
• une couche intérieure - comprenant les polymères Eudragit RS et Eudragit RL, du talc et du citrate d'éthyle ; et
• une couche extérieure - comprenant le polymère Eudragit L-55, du talc et du citrate d'éthyle.

19. La forme galénique pharmaceutique gastrorésistante, selon la revendication 1, dans laquelle la forme dosifiée est en forme de microcapsule incluse :
• soit dans un comprimé à matrice gastrorésistant formant un comprimé ; ou
• dans un comprimé à matrice inerte qui est par la suite enrobé d'une couche d'un polymère gastrorésistant et /ou acidorésistant formant un comprimé ; ou
• dans un comprimé d'un polymère gastrorésistant et / ou acidorésistant ; ou
• dans un comprimé inerte qui est par la suite enrobé d'une couche d'un polymère gastrorésistant et /ou acidorésistant.

20. La forme galénique pharmaceutique gastrorésistante, selon la revendication 19, dans laquelle la forme dosifiée est un comprimé comprenant des microcapsules incluses dans un comprimé à matrice gastrorésistant.

21. La forme galénique pharmaceutique gastrorésistante, selon la revendication 20, dans laquelle le comprimé à matrice est une combinaison de phtalate d'hydroxypropylméthylcellulose avec un mélange d'acétate de polyvinyle et de polyvinylpyrrolidone.

22. La forme galénique pharmaceutique gastrorésistante, selon la revendication 19, dans laquelle le polymère gastrorésistant et/ ou acidorésistant est choisi à partir du groupe composé des dérivés de copolymère d'acide méthacrylique, de phtalate d'hydroxypropylméthylcellulose, de phtalate d'hydroxyéthylcellulose, de phtalate acétylcellulose, de phtalate de polyvinyle acétyle, d'acéto-succinate d'hydroxypropylméthylcellulose ou de leurs combinaisons.
